**Europäisches Patentamt**

**European Patent Office**

**Office européen des brevets**

(19)

(11) Veröffentlichungsnummer : **0 037 381**
**B1**

(12) # EUROPÄISCHE PATENTSCHRIFT

(45) Veröffentlichungstag der Patentschrift :
17.10.84

(51) Int. Cl.³ : **A 61 F 1/00, C 08 J 7/14**

(21) Anmeldenummer : **81810121.4**

(22) Anmeldetag : **26.03.81**

(54) **Verfahren zur Herstellung neuer Organtransplantate.**

(30) Priorität : **31.03.80 CH 2529/80**

(43) Veröffentlichungstag der Anmeldung :
**07.10.81 Patentblatt 81/40**

(45) Bekanntmachung des Hinweises auf die Patenterteilung : **17.10.84 Patentblatt 84/42**

(84) Benannte Vertragsstaaten :
**AT BE CH DE FR GB IT LI NL SE**

(56) Entgegenhaltungen :
**CH-A- 595 105
DE-A- 2 519 107
DE-B- 1 470 805
DE-C- 1 063 330
GB-A- 1 257 263
US-A- 3 093 439
US-A- 3 166 074
Die Akte enthält technische Angaben, die nach dem Eingang der Anmeldung eingereicht wurden und die nicht in dieser Patentschrift enthalten sind.**

(73) Patentinhaber : **Solco Basel AG
Gellertstrasse 18
CH-4052 Basel (CH)**

(72) Erfinder : **Fraefel, Wolfgang, Prof. Dr.
Au Noyeret
CH-1772 Grolley (CH)**
Erfinder : **Lichti, Heinz Felix, Dr.
Spechtweg 9
CH-4125 Riehen (CH)**
Erfinder : **Brunetti, Massimo
Sonnenbergstrasse 22
CH-4127 Birsfelden (CH)**

(74) Vertreter : **Frossard, Michel, Dr. et al
A. Braun, Braun, Héritier, Eschmann AG, Patentanwälte Holbeinstrasse 36-38
CH-4051 Basel (CH)**

Beschreibung

Das Angebot an Prothesenmaterial in der rekonstruktiven Chirurgie für krankhaft veränderte oder in ihrer Funktion beeinträchtigte Organe oder Organteile umfasst mehrere Möglichkeiten (F. Largiader, « Organ Transplantation », 2. Aufl., Georg Thieme Verlag, Stuttgart 1970). Der Ersatz des ursprünglichen Organes oder Organteiles kann nämlich erfolgen :
— durch körpereigenes Material, z. B. autologe Venen [G. E. Mavor und Mitarb., J. Cardiovasc. Surg. 16 (1975), 130]
— durch Implantate aus Kunststoff [M. E. De Bakey und Mitarb., « Fifteen Year's Experience with Dacron Vascular Prostheses », Brochure, Baylor Coll. Med. 1971 (Amer. Coll. Surg. Exhibit. 1971)] oder
— durch entsprechend präparierte Transplantate von verschiedenen Tierarten (P. Walter und H. Schmitz, « Der heterologe Gefässersatz », Editio Cantor, Aulendorf 1976).

Letztere entstammen zur Hauptsache Tierorganen oder Teilen davon (z. B. Kälberkarotiden oder Schweineherzklappen), welche nach der Entnahme durch eine enzymatische Hydrolyse (z. B. mit Ficin, DE-C-1 063 330) von potentiell antigenwirksamen Proteinen befreit werden. Die nach dieser Behandlung verbleibende interzelluläre Matrix besteht zur Hauptsache aus Kollagenfasern vom Typ I. Um diesem nach proteolytischer Hydrolyse erhaltenen Kollagengerüst die für eine Prothese notwendige Dichtigkeit und Stabilität zu verleihen, wird eine sogenannte « Gerbung » durchgeführt. Für diese Gerbung — oder Quervernetzung (cross linking) — von derartigen Kollagenfasern stehen Aldehyde wie Dialdehydstärke (US-A-3 093 439), Glutardialdehyd, Formaldehyd (DE-C-1 063 330) sowie Glyoxal, Polyacrolein, ferner auch Acetaldehyd und Crotonaldehyd zur Verfügung. Das gemeinsame Merkmal dieser Stoffe zur Quervernetzung besteht darin, dass sie mit den ε-Aminogruppen der Lysin-Reste der Kollagen-Peptidkette unter Bildung von Schiff'schen Basen reagieren.

Es war auch schon bekannt (DE-B-1 470 805 und US-A-3 166 074), die Reissfestigkeit von Kollagenfasern und -gebilden und deren Widerstand gegen enzymatische Zersetzung durch Behandlung mit einer einen Aldehyd, ein Aluminiumsalz und 2,4-Dihydroxybenzoesäure enthaltenden Gerblösung zu erhöhen ; derart gegerbte Kollagenfäden und -stränge können in der Chirurgie verwendet werden.

Bei den auf diese Weise vorbehandelten Organen, wie beispielsweise Gefässprothesen (u. a. US-A-3 093 439 oder CH-A 595 105), zeigte sich jedoch, dass durch den enzymatischen Abbau mittels Ficin die ursprünglich vorhandenen biophysikalischen Eigenschaften, wie die Elastizität in axialer und radialer Richtung sowie die äusserst glatte Beschaffenheit der Gefässinnenfläche, vollständig verloren gehen. Als Folge davon konnten bis heute die Erfordernisse für eine ideale Gefässprothese aus biologischem Material nicht erfüllt werden. Dasselbe trifft auch für den Ersatz anderer Organe oder Organteile zu. Wird hingegen der Ficin-Abbau unterlassen, so kann die Gefahr einer Antigenwirkung nicht ausgeschlossen werden, wie z. B. beim Verfahren zum Präparieren von natürlichem Gewebe zur Implantation gemäss DE-A-2 519 107.

Während für ein als Prothese zu verwendendes biologisches Material die Möglichkeit einer Antigenwirkung oder einer sonstigen pharmakologischen Wirkung ausgeschlossen werden muss, sind Verfahren bekannt (GB-A-1 257 263), bei welchen biologisch aktive Proteine — z. B. Enzyme, Antikörper, Antigene, Allergene, Hormone — auf einem Trägermaterial durch ein Vernetzungsmittel gebunden werden. Erhalten werden dadurch Träger-fixierte Proteine mit gleicher biologischer Aktivität wie das Ausgangsprodukt. Zur Vernetzung werden wiederum Dialdehyde, ferner Bisdiazo-benzidin, Bisepoxide, Diisocyanate, Carbodiimide usw. vorgeschlagen, d. h. Verbindungen, welche allesamt keine physiologischen Produkte sind und keine Bindungstypen wie jene der tierischen Gewebe bilden können.

Eine weitere Modifikation am Kollagengerüst besteht darin, dass aliphatische Carbonsäuren durch Acylierung kovalent an die Seitenkette von Aminosäuren unter Bildung von stärker negativ geladenen Derivaten gebunden werden (bekanntlich fördert ein positiver Ladungsüberschuss die Thrombusbildung). Das Verfahren führt aber nicht zu einer Quervernetzung im Kollagengerüst (P. N. Sawyer und Mitarb., « Vascular Grafts », Appleton Century Crofts, New York 1977, S. 282 ff).

Trotz den oben geschilderten Bemühungen lehrt die klinische Erfahrung, dass die bereits vorgeschlagenen Transplantate im allgemeinen, zumindest auf lange Sicht, nicht als voll befriedigend bezeichnet werden können (H. Haimovici, « Vascular Surgery, Principles and Techniques », McGraw-Hill, New York 1976, S. 304).

Es wurde nun überraschenderweise gefunden, dass durch eine neuartige inter- und/oder intramolekulare Quervernetzung der Makromoleküle der interzellulären Matrix von Organen oder Organteilen, vor dem oder sogar auch ohne den proteolytischen Abbau durch Ficin, die ursprünglich vorhandenen biophysikalischen Eigenschaften, wie z. B. bei den Gefässprothesen die axiale und radiale Elastizität sowie die glatte Beschaffenheit der Gefässinnenfläche, weitgehend erhalten bleiben.

Diese Quervernetzung unterscheidet sich von den bekannten Verfahren dadurch, dass sie nicht durch die Bildung von Schiff'schen Basen zustandekommt, sondern durch chemisch wesentlich stabilere Säureamidbindungen zwischen den Aminogruppen resp. durch Esterbindungen der alkoholischen Hydroxylgruppen der Peptidketten der interzellulären Matrix und den Carboxylgruppen einer Di-, Tri- oder Polycarbonsäure, welche als Mittel zur Quervernetzung eingesetzt wird.

Das erfindungsgemässe Verfahren besteht darin, dass man Organe oder Organteile von Fischen, Vögeln oder Säugetieren, vorzugsweise von höheren Säugetieren, einer Quervernetzung der Makromoleküle der interzellulären Matrix durch Bildung von Amidbindungen, resp. Esterbindungen zwischen den Aminogruppen, resp. den alkoholischen Hydroxylgruppen der Peptidketten und den Carboxylgruppen von Di-, Tri- oder Polycarbonsäuren der aliphatischen, cycloaliphatischen, aromatischen oder heterocyclischen Reihe unterwirft.

Obschon nicht erforderlich, kann eine Weiterbehandlung der erhaltenen, durch Amid- und Esterbindungen quervernetzten interzellulären Matrix gemäss folgenden Richtlinien gewisse Vorteile mit sich bringen.

Zunächst kann die Matrix in einer ersten zusätzlichen Verfahrensstufe mit einem Dialdehyd behandelt werden. Die Behandlung mit dem Dialdehyd dient hauptsächlich zur Bindung von bei der Quervernetzung eventuell nicht erfassten Aminogruppen. Dadurch wird der negative Ladungsüberschuss erhöht, welcher erfahrungsgemäss die Gefahr einer Thrombosierung, z. B. von Gefässtransplantaten, herabsetzt (P. N. Sawyer und Mitarb., ibidem).

Anstelle der oben erwähnten Behandlung mit einem Dialdehyd kann die Matrix in einer anderen (alternativen) zusätzlichen Verfahrensstufe durch hydrolytischen Abbau mit Ficin, Papain oder einer anderen Protease von gleicher oder ähnlicher Substratspezifität von potentiell antigenwirksamem Material befreit werden. Durch die zusätzliche Hydrolyse oder Proteolyse werden Organtransplantate erhalten, bei welchen die Möglichkeit einer Antigenwirkung, d. h. der Auslösung einer immunologischen Abwehrreaktion mit Abstossung des Transplantates oder Implantates, auch auf lange Sicht ausgeschlossen ist.

Wird die erwähnte Proteolyse mit Ficin, Papain oder dergl. durchgeführt, so werden in der interzellulären Matrix einzelne Aminogruppen freigesetzt. Um diese und die bei der vorhergegangenen Quervernetzung etwa nicht umgesetzten Aminogruppen zu binden, empfiehlt es sich, die Matrix in einer zweiten zusätzlichen Verfahrensstufe entweder mit einem Dialdehyd zu behandeln — wie oben schon beschrieben wurde — oder sie nochmals einer Quervernetzung durch Bildung von Amidbindungen mit Hilfe einer der erwähnten Di-, Tri- und Polycarbonsäuren zu unterwerfen.

Im folgenden wird die Erfindung ausführlich beschrieben.

Als Ausgangsmaterial werden insbesondere Arterien, Venen, Herzklappen sowie Perikard und dergl. aus Vögeln und höheren Säugetieren geeigneter Grösse verwendet. Als Lieferanten kommen in Frage sowohl der Mensch selber (autologe Transplantate) als auch Kälber, Rinder, Pferde, Schafe, Schweine, Gänse, Truthühner, Fasanen und andere Tiere (Heterologe Transplantate). Bevorzugt werden hierzu Organe und Organteile von Tieren wegen ihrer allgemeineren Verfügbarkeit, besonders aber von jungen Tieren wegen ihrer vorzüglichen Elastizität.

Es versteht sich von selbst, dass die Organe und Organteile sofort nach ihrer Entnahme von den umliegenden Geweben befreit und dass, im Falle von Arterien oder Venen, die Kollateralen durch Ligatur abgebunden werden. Danach werden sie unmittelbar dem Verfahren unterworfen oder bis zur Anwendung des Verfahrens in Wasser, physiologischer Kochsalzlösung oder einer anderen physiologischen wässrigen Lösung, z. B. Tween 80$^R$ (Polyoxyäthylenderivate der Sorbitanoleate), oder in einer nichtwässrigen Flüssigkeit, z. B. Dimethylsulfoxid, gegebenenfalls unter Zusatz von etwas Natriumazid, bei 0 bis − 15 °C aufbewahrt.

Die stets vorhandene Stufe im erfindungsgemässen Verfahren besteht aus einer Quervernetzung der Peptidketten von Bestandteilen der interzellulären Matrix ; sie beruht auf der Verbindung von zwei, drei oder mehreren Aminogruppen (zum überwiegenden Teil ε-Aminogruppen der Lysinradikale), resp. alkoholischen Hydroxylgruppen der Peptidketten über aliphatische, cycloaliphatische, aromatische oder heterocyclische Di-, Tri- bzw. Polycarbonsäuren durch Säureamid- oder Esterbindungen.

Unter den genannten Carbonsäuren eignen sich vor allem solche, die weder Oxogruppen (Aldehyd- und Ketogruppen) noch Aminogruppen aufweisen. Es kommen insbesondere solche Di-, Tri- und Polycarbonsäuren in Frage, welche keine funktionellen Gruppen tragen, ausser den Carboxylgruppen und eventuell Hydroxigruppen.

Bevorzugt werden als aliphatische Dicarbonsäuren und Tricarbonsäuren solche mit bis zu 12 Kohlenstoffatomen, d. h. insbesondere die Oxalsäure, Malonsäure, Bernsteinsäure, Aepfelsäure, Glutarsäure, Adipinsäure, Pimelinsäure, Korksäure, Sebacinsäure und Dodecandisäure, ferner die Weinsäure und die Schleimsäure, und als Tricarbonsäuren die Tricarballylsäure und die Citronensäure.

Unter den cycloaliphatischen Di-, Tri- und Polycarbonsäuren eignen sich unter anderen die Cyclopentandicarbonsäuren und die Cyclohexandicarbonsäuren, z. B. die Cyclohexan-1,4-dicarbonsäure.

Als aromatische Dicarbonsäuren sollen hier insbesondere die Phthalsäure, Isophthalsäure und Terephthalsäure und als aromatische Tricarbonsäuren die Trimesinsäure und die Trimellitsäure genannt werden.

Unter den heterocyclischen Di-, Tri- und Polycarbonsäuren kommen unter anderen in Frage die Furan-2,5-dicarbonsäure, die Tetrahydrofuran-2,5-dicarbonsäure, oxidierte Stärke und Carboxymethylcellulose.

Der zwischen zwei der Vernetzung zugänglichen ε-Aminogruppen bestehende Abstand und die für die Durchführung des Verfahrens zweckmässige Löslichkeit in den Lösungsmitteln lassen bei den erwähnten bevorzugten Gruppen eine engere untere und obere Grenze empfehlenswert erscheinen. Besonders bevorzugt sind also einerseits aliphatische Dicarbonsäuren mit 3 bis 12 Kohlenstoffatomen, d. h. von der Malonsäure bis zur Dodecandisäure. Andererseits haben sich aber auch höhermolekular Polycarbonsäuren, wie beispielsweise oxydierte Stärke, besonders bewährt.

Die Quervernetzung kann aufgrund sämtlicher in der Chemie gebräuchlichen Methoden zur Bildung einer Amidbindung zwischen einer Amino- und einer Carboxylgruppe, bzw. einer Esterbindung zwischen einer Hydroxyl und einer Carboxylgruppe erreicht werden ; siehe dazu u. a. H. D. Law, « The Organic Chemistry of Peptides » (John Wiley & Sons Ltd., London-New York 1970). Es können insbesondere — ohne Anspruch auf Vollständigkeit — die Säurechloride, -azide oder -anhydride der genannten Carbonsäuren mit den freien Amino- und Hydroxylgruppen der interzellulären Matrix umgesetzt werden. Ebenfalls können die freien Di-, Tri- bzw. Polycarbonsäuren mit Hilfe eines geeigneten Kupplungsreagenses, wie die Carbodiimide, z. B. Dicyclohexylcarbodiimid, an die freien Amino- und Hydroxylgruppen der interzellulären Matrix gebunden werden.

Die Reaktion wird in der Regel in einem wasserfreien organischen Lösungsmittel, wie Tetrahydrofuran, Dioxan, Pyridin, Dimethylformamid, Dimethylacetamid, Dimethylsulfoxid und Hexamethylphosphorsäuretriamid, oder in einem Gemisch zweier oder mehrerer derselben, durchgeführt. Wird ein wasserlösliches Kupplungsreagens, z. B. N-Aethyl-N'-(3-dimethylaminopropyl)-carbodiimid-hydrochlorid, eingesetzt, so eignet sich als Lösungsmittel auch Wasser.

Die derart stabilisierten Organe und Organteile können nun in einer (fakultativen) zweiten Stufe des Verfahrens mittels Formaldehyd oder eines Dialdehyds einer Gerbung oder Quervernetzung unterzogen werden, bei welcher die möglicherweise noch freien Aminogruppen der Kollagen-Peptidkette unter Bildung von Schiff'schen Basen umgesetzt werden. Diese Behandlung ist auch insofern von Vorteil, als insbesondere die mit Glutardialdehyd erhaltenen Prothesen bereits praktisch steril sind.

Als Dialdehyd eignet sich im Prinzip jeder Dialdehyd ; bevorzugt werden Dialdehydstärke und vor allem Glutardialdehyd. Die Reaktion mit dem Dialdehyd wird mit Vorteil in wässeriger Lösung, beispielsweise nach der Methode gemäss CH-Patent 595 105 oder US-Patent 3 093 439 durchgeführt.

Alternativ können die durch Quervernetzung stabilisierten Organe oder Organteile gewünschtenfalls der proteolytischen Wirkung eines geeigneten Enzyms ausgesetzt werden, ohne wesentliche Einbusse ihrer ursprünglich vorhandenen biophysikalischen und biochemischen Eigenschaften. Dieses Verhalten ist darauf zurückzuführen, dass die Peptidketten des Kollagens durch die Quervernetzung fixiert werden, und die stabilisierenden Elemente der interzellulären Matrix, wie Elastin, Proteoglykane sowie Struktur-Glykoproteine, durch eine ähnliche Blockierung ihrer Amino- und Hydroxylgruppen gegen die Hydrolyse beständig gemacht werden.

Die bevorzugte zweite Stufe des Verfahrens besteht also aus einer Inkubation des Organmaterials in einer zweckmässigerweise wässerigen Lösung von Ficin, Papain oder einer anderen Protease von gleicher oder ähnlicher Substratspezifität. Die Hydrolyse oder Proteolyse erfolgt vorzugsweise mit Ficin, mit Vorteil im pH-Bereich von 4,0 bis 5,5 ; sie kann u. a. nach Beispiel 3 von CH-Patent 595 105 durchgeführt werden.

Die auf die Hydrolyse mit Vorteil folgende nochmalige Quervernetzung kann mit einem Dialdehyd oder einer der oben genannten Di-, Tri- und Polycarbonsäuren durchgeführt werden ; es eignen sich dazu die oben beschriebenen Methoden.

Die erhaltenen Organtransplantate werden anschliessend mit Wasser gründlich gewaschen, sterilisiert und bis zu ihrer Anwendung in zugeschweissten Kunststoffbeuteln aufbewahrt. Die Sterilisation der Produkte erfolgt ausschliesslich auf chemischem Weg mit Hilfe von Propen-1,2-oxid (siehe im experimentellen Teil unter K-I-Lösung), Propiolakton (β-Hydroxy-propionsäurelakton) oder Aethylenoxid.

Zusammenfassend werden durch das erfingungsgemässe Verfahren Prothesen erhalten, die sich als Ersatz defekter Organe oder Teile davon besonders gut eignen, weil
— sich ihre inter- bzw. intramolekularen Querbindungen (Säureamid-, resp. Esterbindungen) durch besondere chemische Stabilität auszeichnen,
— aufgrund der erwähnten Art der Quervernetzung, vor dem oder auch ohne den proteolytischen Abbau, ihre biophysikalischen (Elastizität, Rigidität) sowie ihre biochemischen (verminderte Thrombogenizität) Eigenschaften und diejenigen des nativen Materials einander äusserst gleichen,
— und sie trotzdem keine Abstossungsreaktionen hervorrufen, d. h. nicht antigen wirken können.

Die Vorteile der erwähnten Eigenheiten liegen für die chirurgische Anwendung und den Heilerfolg auf der Hand, und sie sind auch in der Tat im Tierversuch bereits bestätigt worden.

Für die Versuche wurden Bastardhunde von 20 bis 25 kg Körpergewicht und die nach Beispiel 9 hergestellten Arterientransplantate verwendet. Die Implantation der Prothesen erfolgte (a) an den beiden Arteriae femorales eines jeden Hundes, unmittelbar distal des Leistenbandes nach der Methode von P. Walter und Mitarb. [Helv. chir. Acta 46 (1979), 81 ff], (b) an den beiden Carotiden, (c) an der Aorta abdominalis, caudal der Abgänge der Arteriae renales [P. Walter und H. Schmitz, Der heterologe Gefässersatz, S. 30, Editio Cantor, Aulendorf 1976], sowie (d) an der infrarenalen Vena cava inferior nach der Methode von S. Horsch u. Mitarb. [Langenbecks Arch. Chir. 344 (1978), 225 ff]. Zum Vergleich wurden parallele Versuche mit Prothesen aus Teflon (R) und Prothesen aus Dacron (R), sowie mit Umbilicalvenen, welche nur mit Glutardialdehyd gegerbt worden waren, durchgeführt. Gemessen wurde die Verschlussrate der Implantate nach sechs Wochen.

Es soll hier hervorgehoben werden, dass besonders die Implantationsstelle a (siehe oben) am Hüftgelenk des Hundes einer hohen mechanischen Beanspruchung (Flexion), die sich zeitweise zu einer Dauerbeanspruchung steigert, unterworfen ist. Mit anderen Worten entspricht diese Versuchsanordnung extrem ungünstigen physiologischen Bedingungen.

Umso aufschlussreicher waren denn auch die Ergebnisse. Die Implantate aus Teflon (R) und der Umbilicalvene ergaben eine Verschlussrate von 100 %, die Implantate aus Dacron (R) eine solche von 77 %, während die Implantate gemäss Beispiel 9 eine Verschlussrate von 50 % zeigten. Die bei den Vergleichsversuchen erhobenen Werte sind statistisch signifikant.

In mittelfristigen, d. h. eine Zeitspanne von 6 Monaten umfassenden Versuchen unter physiologisch günstigeren Verhältnissen, namentlich an der Arteria carotis, sank die Verschlussrate der Implantate gemäss Beispiel 9 auf 20 % ; der ermittelte Wert ist statistisch signifikant. Bei Versuchen im infrarenalen Bereich wurde mittelfristig (6 Monate) keine Thrombosierung beobachtet.

Vergleichsversuche am Menschen laufen zur Zeit in einer Universitätsklinik.

Beispiel 1

Kälberkarotiden werden vom umgebenden Bindegewebe befreit und die Kollateralen abgebunden. Nach der mechanischen Präparation werden sie mit entionisiertem Wasser gewaschen, mit Fliesspapier abgetrocknet, auf einen Glasstab von z. B. 3 mm Durchmesser aufgezogen und zur Entwässerung in einen mit Tetrahydrofuran gefüllten Messzylinder gestellt. In Abständen von 1 bis 2 Stunden wird der Inhalt des Messzylinders umgeschwenkt. Nach 5 Stunden wird die Flüssigkeit durch neues Tetrahydrofuran ersetzt. Nach weiteren 2 bis 3 Stunden werden die Glasstäbe entfernt.

Am nächsten Tag werden die Arterien in eine 2 %-ige (Gew./Vol.) Lösung von Adipinsäurechlorid in Tetrahydrofuran gebracht und darin während 24 Stunden belassen. Die Lösung wird gelegentlich umgeschwenkt oder mit einer Pumpe umgewälzt. Danach werden die Arterien in reines Tetrahydrofuran, anschliessend in Tetrahydrofuran-Wasser (1 : 1 Vol./Vol.) und schliesslich in Phosphat-gepufferte Kochsalzlösung eingelegt und jeweils 30 bis 60 Minuten darin belassen. Die Arterien sind nun bereit zur Sterilisation oder zur Hydrolyse mit Ficin, Papain oder dergl.

a) Sterilisation mit Propen-1,2-oxid

Die Arterien werden während 16 bis 24 Stunden, eventuell auch länger, in eine 1 %-ige (Gew./Vol.) Lösung von Propen-1,2-oxid in Aethanol-Wasser-(1 : 1) (Vol./Vol.) gelegt. Anschliessend werden sie unter sterilen Bedingungen in sterile PBS-Lösung eingelegt und mit dieser zusammen in sterile Kunststoffbeutel eingeschweisst.

PBS-Lösung

In 40 Litern Wasser werden gelöst 320 g Natriumchlorid, 8 g Kaliumchlorid, 51,2 g Dinatriumhydrogenphosphat-dihydrat, 8,0 g Kaliumdihydrogenphosphat, 5,2 g Calciumchloriddihydrat und 4,0 g Magnesiumchlorid-hexahydrat.

Will man die Arterien in Aethanol-Wasser-Gemisch aufbewahren, so genügt es, sie in die oben erwähnte alkoholisch-wässrige Propenoxid-Lösung zu legen und mit der Flüssigkeit zusammen in Kunststoffbeutel einzuschweissen.

b) Sterilisation mit Propiolakton (β-Hydroxypropionsäurelakton

Die folgenden Salze werden in ca. 900 ml entionisiertem Wasser aufgelöst : 0,236 g Natriumchlorid, 0,248 g Kaliumchlorid, 0,363 g Calciumchlorid-dihydrat, 0,190 g Magnesiumchlorid-hexahydrat und 0,172 g Kaliumdihydrogenphosphat. Mit etwas 0,1-N wässriger Natronlauge wird der pH-Wert der Lösung auf 7,4 angehoben. Anschliessend werden 11,782 g Natriumhydrogencarbonat in der Lösung aufgelöst und das Volumen durch Zugabe von Wasser auf 1 000 ml ergänzt. Unmittelbar vor der Sterilisation der Arterien gibt man zu 1 Liter dieser Lösung 8,86 ml (10,08 g) Propiolakton, legt die Arterien in die Mischung und schweisst sie mitsamt der Flüssigkeit in einem Kunststoffbeutel ein.

c) Sterilisation mit Aethylenoxid

Die in PBS-Lösung oder physiologische Kochsalzlösung eingelegten Arterien werden mitsamt der Flüssigkeit in einem Kunststoffbeutel eingeschweisst. Man sterilisiert, indem man den verschlossenen Kunststoffbeutel nach bekannter Methode in einem Sterilisationsapparat mit Aethylenoxid während 4 Stunden bei 25 bis 30 °C begast.

Beispiel 2

Vier mechanisch präparierte Kalbsarterien werden einzeln auf 4 mm dicke Glasstäbe aufgezogen und in einen mit Pyridin gefüllten Messzylinder zu 500 ml gestellt. Eine Stunde später sind die Arterien so steif, dass die Glasstäbe weggenommen werden können. Die Flüssigkeit wird abgegossen und durch neues Pyridin ersetzt. Nach einer Stunde wird das Pyridin nochmals erneuert. Eine weitere Stunde später werden die Arterien in ein Gemisch eingebracht, das folgendermassen hergestellt worden ist ; in ein Gemisch aus 98 ml Pyridin und 2 ml Dimethylformamid spritzt man unter Rühren 2 ml (2,5 g) Adipinsäurechlorid ein ; dabei bildet sich ein feiner Niederschlag von Adipylpyridiniumchlorid. Wenn er sich abgesetzt hat, wird er mit einer Siebplatte aus Porzellan abgedeckt. Dann stellt man die Arterien auf die Siebplatte, so dass sie ganz von der Flüssigkeit bedeckt werden, mit dem Niederschlag aber nicht in Berührung kommen. 18 Stunden später werden die Arterien aus der Flüssigkeit herausgenommen und für 30 Minuten in K-I-Lösung (siehe Beispiel 9, nachfolgend) getaucht. Dann wäscht man sie viermal mit 0,05-N steriler wässriger Essigsäure und zweimal mit sterilem Phosphatpuffer (1/15-molar) von pH 8. Zum Schluss werden sie in sterile PBS-Lösung eingelegt und mit dieser Flüssigkeit zusammen in einen Kunststoffbeutel eingeschweisst.

Beispiel 3

10 Kalbsarterien werden mechanisch präpariert und für drei Stunden senkrecht in einen mit Tetrahydrofuran gefüllten Messzylinder zu 500 ml gestellt. Dann stellt man sie einzeln in ca. 2 cm weite Glasrohre und überschüttet sie mit je 40 ml einer 0,06 %-igen (Gew./Vol.) Lösung von Adipinsäure in Tetrahydrofuran-Wasser-(4 : 1) (Vol./Vol.). Nach einer Stunde werden je 40 ml einer 2 %-igen (Gew./Vol.) Lösung von Dicyclohexylcarbodiimid in Tetrahydrofuran hinzugefügt und durch Schütteln und Schwenken mit der bereits vorhandenen Lösung vermischt. Nach 3 Stunden legt man die Arterien in K-I-Lösung (siehe Beispiel 9, nachfolgend) ein. Am folgenden Tag wäscht man sie mit je 40 ml sterilem Aethanol-Wasser-(1 : 1) (Vol./Vol.), sterilem Phosphatpuffer (1/15-molar) von pH 8 und legt sie in sterile PBS-Lösung ein. Zum Schluss werden sie zusammen mit der PBS-Lösung in Kunststoffbeutel eingeschweisst.

Beispiel 4

10 Kalbsarterien werden nach der mechanischen Präparation in einen mit Dimethylformamid gefüllten Messzylinder zu 500 ml gestellt. Nach 3 Stunden stellt man sie einzeln in Glasrohre von ca. 2 cm innerem Durchmesser und übergiesst sie mit je 40 ml einer 0,06 %-igen (Gew./Vol.) Lösung von Adipinsäure in Dimethylformamid-Wasser-(4 : 1). Nach einer Stunde werden je 40 ml einer 2 %-igen (Vol./Vol.) Lösung von Dicyclohexylcarbodiimid in Dimethylformamid zugegeben und mit der bereits eingefüllten Adipinsäure-Lösung durch Schütteln und Schwenken vermischt. 3 Stunden später wird die Flüssigkeit aus jedem Rohr abgegossen und durch je 80 ml K-I-Lösung ersetzt. Am folgenden Tage wäscht man die Arterien mit je 40 ml sterilem Aethanol-Wasser-(1 : 1) (Vol./Vol.) und sterilem Phosphatpuffer (1/15-molar) von pH 8. Schliesslich werden sie in sterile PBS-Lösung eingelegt und mit dieser zusammen in einem Kunststoffbeutel eingeschweisst.

Beispiel 5

4 Kalbsarterien werden in der gewohnten Weise vom Bindegewebe befreit und die Kollateralen abgebunden. Sie werden anschliessend für 2 Stunden in 60 ml 0,02 %-ige (Gew./Vol.) wässrige Adipinsäure-Lösung eingelegt. Dann kommen sie in 60 ml einer wässrigen Lösung, die Adipinsäure in einer Konzentration von 0,2 g pro Liter und N-Aethyl-N'-(3-dimethylaminopropyl)-carbodiimid-hydrochlorid in einer Konzentration von 25 g pro Liter enthält. Nach 20 Stunden werden die Arterien in K-I-Lösung eingelegt, 24 Stunden später in sterile PBS-Lösung. Mit dieser zusammen werden sie in sterile Kunststoffbeutel eingeschweisst.

Beispiel 6

Mechanisch präparierte Arterien legt man für 2 Stunden in 60 ml einer Lösung von 0,1 % (Gew./Vol.) Korksäure in Wasser, nachher in 60 ml einer wässrigen Lösung, die pro Liter 0,24 g Korksäure und 25 g N-Aethyl-N'-(3-dimethylaminopropyl)-carbodiimid-hydrochlorid enthält. Nach 20 Stunden werden die Arterien in K-I-Lösung, nach weiteren 24 Stunden in sterile PBS-Lösung eingelegt und mit dieser zusammen in sterile Kunststoffbeutel eingeschweisst.

Beispiel 7

Nach der in Beispiel 1 beschriebenen Arbeitsweise werden Kälberkarotiden mit Adipinsäurechlorid in Tetrahydrofuran behandelt. Die erhaltenen Arterien werden anschliessend nicht sterilisiert, sondern mit Ficin folgendermassen hydrolysiert :

1 Liter Wasser wird auf 37 °C erwärmt. 10 g Ficin werden unter Rühren zugegeben ; das Ficin löst sich unter Umständen nur teilweise auf. Anschliessend wird 1 g Cystein unter Rühren zugegeben und aufgelöst. Mit 1-N wässriger Trinatriumcitrat-Lösung wird der pH-Wert der Lösung auf 6,0 angehoben. Ist die Flüssigkeit trübe, wird sie durch ein Baumwolltuch filtriert und auf diese Weise von der Trübung befreit. Dabei sinkt ihre Temperatur auf ca. 25 bis 30 °C.

Die Arterien werden in einen Messzylinder gestellt und mit der klaren Ficin-Lösung übergossen. Der Messzylinder wird in ein Wasserbad von 37 bis 38 °C gestellt, damit sich sein Inhalt auf ca. 37 °C erwärmt. Spätestens nach 3 Stunden bei 37 °C ist die Reaktion zu Ende. Jetzt wird die Ficin-Lösung abgegossen und verworfen. Die Arterien werden eine Viertelstunde lang mit fliessendem entionisiertem Wasser von ca. 20 °C gewaschen. Dann wird das Wasser abgegossen. Die Arterien befinden sich noch immer im Messzylinder. Sie werden mit 1 Liter wässriger Lösung übergossen, die 11,9 g Natriumchlorit (NaClO$_2$) enthält. Nach 18 Stunden bei Zimmertemperatur wird die Flüssigkeit abdekantiert, und die Arterien werden etwa eine Viertelstunde lang mit fliessendem entionisiertem Wasser von ca. 20 °C gewaschen. Sie sind jetzt bereit zur Sterilisation oder zur Nachbehandlung mit einem geeigneten Aldehyd.

Beispiel 8

Nach der in Beispiel 1 beschriebenen Arbeitsweise werden Kälberkarotiden mit Adipinsäurechlorid in Tetrahydrofuran behandelt. Die erhaltenen Arterien werden anschliessend nicht sterilisiert, sondern mit Ficin folgendermassen hydrolysiert :

1 Liter Citronensäure-Phosphat-Pufferlösung von pH 4,5, hergestellt nach T. C. McIlvaine [J. Biol. Chem. 49, 183 (1921)], wird vorgelegt. 10 g Ficin werden unter Rühren zugegeben und aufgelöst. Anschliessend wird 1 g Cystein unter Rühren zugegeben und aufgelöst.

Die Arterien werden in einen Messzylinder gestellt und mit der oben beschriebenen Ficin-Lösung übergossen. Die weitere Behandlung erfolgt nach der Methode von Beispiel 7.

Beispiel 9

Kälberkarotiden werden, wie in Beispiel 7 beschrieben wird, zuerst mit Adipinsäurechlorid in Tetrahydrofuran quervernetzt und hierauf einer Hydrolyse mit Ficin unterworfen.

Anschliessend werden sie erneut auf Glasstäbe aufgezogen und in eine 4 %-ige, wässrige Glutardialdehyd-Lösung gestellt. Nach 24 Stunden werden die Stäbe entfernt und die Arterien während mindestens 90 Min. in fliessendem Wasser gewaschen. Zum Schluss werden sie in K-I-Lösung eingelegt und in einen Kunststoffbeutel eingeschweisst.

K-I-Lösung

720 ml Wasser und 720 ml Aethanol werden vermischt. Dieser Lösung werden 16,9 ml (14,1 g) Propen-1,2-oxid zugesetzt. Diese Lösung wird immer frisch zubereitet und unmittelbar nach der Herstellung zum Sterilisieren benützt.

Beispiel 10

7 Kalbs-Herzbeutel werden vom Fett befreit, weitgehend glatt gestrichen und in einer flachen Schale in 500 ml Tetrahydrofuran eingelegt. Die Flüssigkeit wird nach 1 und nach 2 Tagen durch neues Tetrahydrofuran ersetzt. Nach insgesamt 4 Tagen werden die Herzbeutel in 500 ml einer 2 %-igen (Gew./Vol.) Lösung von Adipinsäurechlorid in Tetrahydrofuran eingelegt und für 1 Tag darin belassen. Dann wird die Flüssigkeit abgegossen. Die Herzbeutel werden dreimal für je eine halbe Stunde in Aethanol-Wasser (1 : 1 Vol./Vol.) eingelegt und anschliessend während einer halben Stunde im fliessenden Wasser gewaschen. Nachher legt man sie in Phosphat-gepufferte Kochsalzlösung von pH 4,5 ein. Sie sind jetzt bereit für die Hydrolyse mit Ficin, Papain oder einem anderen geeigneten Enzym.

Die mit Adipinsäurechlorid vernetzten Herzbeutel werden in eine Ficin-Lösung getaucht, wie sie im Beispiel 7 oder 8 beschrieben wird. Die Temperatur beträgt 37 °C. Nach 3 Stunden wird die Ficin-Lösung abgegossen, und die Herzbeutel werden während einer Viertelstunde mit fliessendem entionisiertem Wasser gewaschen. Dann werden sie in eine wässrige Lösung gelegt, die pro Liter 11,9 g Natriumchlorit enthält. 18 Stunden später wird die Lösung abgegossen. Die Herzbeutel werden während einer Viertelstunde mit fliessendem entionisiertem Wasser ausgewaschen. Sie sind jetzt zur Nachbehandlung mit einem geeigneten Aldehyd oder zur Sterilisation bereit.

Die mit Adipinsäurechlorid vernetzten und mit Ficin hydrolysierten Herzbeutel werden für 24 Stunden in eine 1 %-ige (Gew./Vol.) wässrige Lösung von Glutardialdehyd eingelegt, durch zweistündiges Waschen im fliessenden Wasser vom Ueberschuss an Aldehyd befreit und entweder mit K-I-Lösung oder mit Aethylenoxid sterilisiert.

a) Sterilisation mit K-I-Lösung

Die oben erwähnten, zum Schluss mit Glutardialdehyd behandelten und ausgewaschenen Herzbeutel werden in K-I-Lösung eingelegt und mit dieser zusammen in einem Kunststoffbeutel eingeschweisst.

b) Sterilisation mit Aethylenoxid

Die oben erwähnten, zum Schluss mit Glutardialdehyd behandelten und ausgewaschenen Herzbeutel werden mit 0,9 %-iger Kochsalzlösung zusammen in je einem Kunststoffbeutel eingeschweisst. Die verschlossenen Beutel werden in einem Sterilisationapparat während 4 Stunden bei 25 bis 30 °C mit Aethylenoxid begast.

Beispiel 11

Kälberkarotiden werden, wie im Beispiel 1 beschrieben, mechanisch präpariert und mit Wasser gewaschen. Man legt sie sodann in 200 ml Tetrahydrofuran ein und ersetzt diese Flüssigkeit nach 1, 2 und 4 Tagen durch neues Tetrahydrofuran. Nach insgesamt 7 Tagen legt man die Arterien in eine 3,2 %-ige (Gew./Vol.) Lösung von Dodecandisäure-dichlorid in Tetrahydrofuran ein und lässt sie während 48 Stunden darin liegen. Danach werden sie für 16 Stunden in Tetrahydrofuran, für 8 Stunden in Tetrahydrofuran-Pufferlösung (1 : 1 Vol./Vol.) von pH 4,5 und schliesslich für einen Tag in wässrige Pufferlösung von pH 4,5 eingelegt. Sie sind jetzt bereit zur Sterilisation oder zur Hydrolyse mit Ficin.

Beispiel 12

Kälberkarotiden werden, wie im Beispiel 7 oder 8 beschrieben, zuerst mit Adipinsäurechlorid in Tetrahydrofuran quervernetzt und anschliessend der Hydrolyse mit Ficin unterworfen. Nach Auswaschen der Natriumchlorit-Lösung wie im Beispiel 7, werden sie in 0,5 %-ige wässrige Glutardialdehyd-Lösung eingelegt. Nach 48 Stunden wird die Flüssigkeit abgegossen. Die Arterien werden hierauf während einer halben Stunde mit fliessendem Wasser gewaschen. Zum Schluss legt man sie in 0,9 %-ige Natriumchlorid-Lösung ein, füllt sie zusammen mit dieser Flüssigkeit in Kunststoffbeutel ein und verschweisst diese. Die verschlossenen Kunststoffbeutel werden, wie im Beispiel 1 unter Abschnitt c) beschrieben, durch Begasen mit Aethylenoxid sterilisiert.

Beispiel 13

550 g Maisstärke werden in 3 Liter Wasser eingetragen und während einer Viertelstunde gerührt. Dann tropft man innerhalb 1 Stunde eine Lösung von 705 g Natriummetaperjodat in 9 Liter Wasser zu. Die Suspension wird 18 Stunden lang weitergerührt. Sie wird dann durch Papier filtriert. Der Rückstand auf dem Filter wird in 1,5 Liter Wasser aufgeschlämmt und wieder abfiltriert. Diesen Prozess des Aufschlämmens und Abfiltrierens wiederholt man fünfmal. Der Rückstand auf dem Filter, noch feucht, wird anschliessend in 3 Liter Aceton eingetragen, während einer halben Stunde kräftig gerührt und anschliessend abfiltriert. Den Filterrückstand trocknet man 42 Stunden lang bei 40 °C im Vakuum. Nachher mahlt man ihn mit einer Kugelmühle zu einem feinen Pulver. 13 g des feinen Pulvers werden in 1 Liter entionisiertem Wasser aufgeschlämmt. Durch Zugabe von gesättigter Natriumhydrogencarbonat-Lösung wird der pH-Wert auf 8,80 eingestellt.

Dann werden Arterien, die — wie im Beispiel 7 oder 8 beschrieben — mit Adipinsäurechlorid quervernetzt und mit Ficin hydrolysiert worden sind, in die oben erhalgene Suspension von Aldehydstärke eingelegt. Die Flüssigkeit wird mit Hilfe eines Vibrators in ständiger Bewegung gehalten. Nach 24 Stunden wird die Flüssigkeit abgegossen. Die Arterien werden 30 Minuten lang im fliessenden Wasser gewaschen. Dann legt man sie in PBS-Lösung ein, schweisst sie mit dieser Flüssigkeit zusammen in einem Kunststoffbeutel ein und sterilisiert sie durch Begasen mit Aethylenoxid.

Beispiel 14

550 g Maisstärke werden zu 3 Liter Wasser gegeben und während einer Viertelstunde gerührt. Dann tropft man innerhalb 1 Stunde eine Lösung von 705 g Natriummetaperiodat in 9 Liter Wasser zu. Die Suspension wird 18 Stunden lang weitergerührt. Sie wird dann durch Papier filtriert. Der Rückstand auf dem Filter wird in 1,5 Liter Wasser aufgeschlämmt und abfiltriert. Den Prozess des Aufschlämmens und Abfiltrierens wiederholt man fünfmal.

Nachher wird der Rückstand in 3 Liter Wasser aufgeschlämmt. Unter ständigem Rühren tropft man innerhalb 1 Stunde eine Lösung von 695 g Kaliumpermanganat in 12 Liter Wasser zu. Die Suspension wird 18 Stunden lang weitergerührt. Dann lässt man das Gemisch 2 Stunden lang stehen. In dieser Zeit setzt sich ein grosser Teil des gebildeten Braunsteins ab. Die überstehende Suspension wird mit Hilfe eines Hebers vorsichtig in ein anderes Gefäss übergeführt; der Bodensatz wird verworfen.

Die Suspension wird über Papier filtriert. Der Rückstand auf dem Filter wird in 1,2 Liter Wasser aufgeschlämmt und abfiltriert. Der Prozess des Aufschlämmens und Abfiltrierens wird dreimal wiederholt. Dann wird der Filterrückstand in 9 Liter 0,5-N Salzsäure von 0 °C aufgenommen und 1 Stunde lang gerührt. Die Suspension wird anschliessend durch Papier filtriert. Der Rückstand auf dem Filter wird in 1,2 Liter Wasser aufgeschlämmt und abfiltriert. Den Prozess des Aufschlämmens und Abfiltrierens wiederholt man dreimal. Der Filterrückstand wird anschliessend in 3 Liter Aceton suspendiert, eine halbe Stunde lang gerührt und abfiltriert. Man trocknet den Filterrückstand 42 Stunden lang bei 40 °C im Vakuum und mahlt ihn nachher mit einer Kugelmühle zu einem feinen Pulver.

2 g dieses Pulvers, welches aus oxidierter, eine Vielzahl von Carboxylgruppen enthaltender Stärke besteht, werden in 1 Liter Wasser suspendiert. Mechanisch präparierte Arterien werden für 2 Stunden in 150 ml Suspension eingelegt. Die flüssigkeit wird mit Hilfe eines Vibrators leicht bewegt. Dann bringt man die Arterien in 200 ml einer Suspension, die pro Liter 0,26 g des oben erwähnten Pulvers sowie 25 g N-Aethyl-N'-(3-dimethylaminorpopyl)-carbodiimid-hydrochlorid enthält. Die Suspension wird andauernd gelinde vibriert. Nach 24 Stunden wird die Flüssigkeit abgegossen. Die Arterien wäscht man eine halbe Stunde lang unter dem fliessenden Wasser. Anschliessend legt man sie in 150 ml Citronensäure-Phosphat-Pufferlösung von pH 4,5 ein und unterwirft sie der im Beispiel 8 beschriebenen Ficin-Behandlung.

**Ansprüche**

1. Verfahren zur Herstellung neuer Organtransplantate aus Organen und Organteilen von Fischen, Vögeln und Säugetieren, dadurch gekennzeichnet, dass man besagte Organe oder Organteile einer Quervernetzung der Makromoleküle der interzellulären Matrix durch Bildung von Amindbindungen zwischen den Aminogruppen der Peptidketten, resp. von Esterbindungen zwischen den Hydroxylgruppen der Peptidketten, und den Carboxylgruppen von Dicarbonsäuren, Tricarbonsäuren oder Polycarbonsäuren der aliphatischen, cycloaliphatischen, aromatischen oder heterocyclischen Reihe unterwirft.

2. Verfahren nach Anspruch 1, dadurch gekennzeichnet, dass Di-, Tri- oder Polycarbonsäuren verwendet werden, welche weder Aldehyd- oder Ketogruppen, noch Aminogruppen aufweisen, insbesondere solche, die nur Carboxylgruppen und eventuell Hydroxigruppen tragen.

3. Verfahren nach Anspruch 2, dadurch gekennzeichnet, dass als aliphatische Carbonsäuren solche mit 3 bis 12 Kohlenstoffatomen, als cycloaliphatische Carbonsäuren die Cyclopentan- und Cyclohexandicarbonsäuren, als aromatische Carbonsäuren die Phthalsäure, Isophthalsäure und Terephthalsäure, die Trimesinsäure und die Trimellitsäure und als heterocyclische Carbonsäuren die Furan- und Tetrahydrofuran-2,5-dicarbonsäure, oxidierte Stärke und Carboxymethylcellulose verwendet werden.

4. Verfahren nach einem der Ansprüche 1 bis 3, dadurch gekennzeichnet, dass man die erhaltene, durch Amid- und Esterbindungen quervernetzte interzelluläre Matrix zusätzlich mit Formaldehyd oder einem Dialdehyd behandelt.

5. Verfahren nach Anspruch 4, dadurch gekennzeichnet, dass als Dialdehyd Dialdehydstärke oder Glutardialdehyd, vorzugsweise letzterer, verwendet wird.

6. Verfahren nach einem der Ansprüche 1 bis 3, dadurch gekennzeichnet, dass man in der erhaltenen, durch Amid- und Esterbindungen quervernetzten interzellulären Matrix zusätzlich, durch Hydrolyse mit Ficin, Papain oder einer anderen Protease von gleicher oder ähnlicher Substratsspezifität, potentiell antigenwirksames Material abbaut.

7. Verfahren nach Anspruch 6, dadurch gekennzeichnet, dass die Hydrolyse mit Ficin durchgeführt wird.

8. Verfahren nach einem der Ansprüche 6 oder 7, dadurch gekennzeichnet, dass man die erhaltene, durch Amid- und Esterbindungen quervernetzte und antigenfreie Matrix zusätzlich mit einem Dialdehyd behandelt.

9. Verfahren nach Anspruch 6 oder 7, dadurch gekennzeichnet, dass man die erhaltene, durch Amid- und Esterbindungen quervernetzte und antigenfreie Matrix zusätzlich einer nochmaligen Quervernetzung durch Bildung von Amidbindungen zwischen freien Aminogruppen und den Carboxylgruppen von Dicarbonsäuren, Tricarbonsäuren oder Polycarbonsäuren der aliphatischen, cycloaliphatischen, aromatischen oder heterocyclischen Reihe unterwirft.

## Claims

1. A method of making organic grafts from organs and organ parts of fishes, birds and mammals, which comprises submitting said organs or organ parts to a crosslinking of the macromolecules of the intercellular matrix by the formation of amide linkages between the amino radicals of the peptide chains, respectively of ester linkages between the hydroxy radicals of the peptide chains, and the carboxyl radicals of dicarboxylic acids, tricarboxylic acids or polycarboxylic acids of the aliphatic, cycloaliphatic, aromatic or heterocyclic series.

2. A method according to claim 1, which comprises using di-, tri- or polycarboxylic acids which do not contain aldehydo or keto radicals, nor amino radicals and especially the ones which contain only carboxyl radicals and optionally hydroxy radicals.

3. A method according to claim 2, which comprises using as aliphatic carboxylic acids those having 3 to 12 carbon atoms, as cycloaliphatic carboxylic acids the cyclopentane and cyclohexane dicarboxylic acids, as aromatic carboxylic acids phthalic acid, isophthalic acid and terephthalic acid, trimesic acid and trimellitic acid, and as heterocyclic carboxylic acids furane- and tetrahydrofurane-2,5-dicarboxylic acid, oxidized starch and carboxymethylcellulose.

4. A method according to any one of claims 1 to 3, which comprises additionally treating the obtained intercellular matrix which is crosslinked through amide and ester linkages, with formaldehyde or a dialdehyde.

5. A method according to claim 4, which comprises using as a dialdehyde dialdehydo-starch or glutardialdehyde, preferably the last one.

6. A method according to any one of claims 1 to 3, which comprises removing potentially antigenic material from the obtained intercellular matrix which is crosslinked through amide and ester linkages, by hydrolysis with ficin, papain or an other protease of same or similar substrate specificity.

7. A method according to claim 6, which comprises carrying out the hydrolysis by means of ficin.

8. A method according to any one of claims 6 and 7, which comprises additionally treating the obtained matrix which is crosslinked through amide and ester linkages and is free of antigens, with a dialdehyde.

9. A method according to claims 6 or 7, which comprises additionally submitting the obtained matrix which is crosslinked through amide and ester linkages and is free of antigens, to another crosslinking by the formation of amide linkages between free amino group and the carboxyl groups of dicarboxylic acids, tricarboxylic acids or polycarboxylic acids of the aliphatic, cycloaliphatic, aromatic and heterocyclic series.

## Revendications

1. Procédé de préparation de nouveaux transplants d'organes à partir d'organes et parties d'organes de poissons, d'oiseaux et de mammifères, caractérisé en ce qu'on soumet lesdits organes ou parties d'organes à une réticulation des macromolécules de la matrice intercellulaire par formation de liaisons amide entre les radicaux amino des chaînes peptidiques ou bien de liaisons ester entre les radicaux hydroxyle des chaînes peptidiques et les radicaux carboxyle d'acides dicarboxyliques, d'acides tricarboxyliques ou d'acides polycarboxyliques de la série aliphatique, cycloaliphatique, aromatique ou hétérocyclique.

2. Procédé suivant la revendication 1, caractérisé en ce qu'on utilise des acides di-, tri- ou polycarboxyliques ne contenant ni radicaux aldéhyde ou céto ni radicaux amino et en particulier ceux ne contenant que des radicaux carboxyle et éventuellement hydroxyle.

3. Procédé suivant la revendication 2, caractérisé en ce qu'on utilise comme acides carboxyliques aliphatiques ceux de 3 à 12 atomes de carbone, comme acides carboxyliques cycloaliphatiques, les acides cyclopentane- et cyclohexanedicarboxyliques, comme acides carboxyliques aromatiques, les acides phtalique, isophtalique et téréphtalique, l'acide trimésique et l'acide trimellitique et comme acides carboxyliques hétérocycliques, les acides furanne- et tétrahydrofuranne-2,5-dicarboxyliques, les amidons oxydés et la carboxyméthylcellulose.

4. Procédé suivant l'une quelconque des revendications 1 à 3, caractérisé en ce qu'on traite en outre au moyen de formaldéhyde ou d'un dialdéhyde la matrice intercellulaire obtenue, réticulée par des liaisons amide et ester.

5. Procédé suivant la revendication 4, caractérisé en ce qu'on utilise comme dialdéhyde, le dialdéhydo-amidon ou le glutaraldéhyde, de préférence ce dernier.

6. Procédé suivant l'une quelconque des revendications 1 à 3, caractérisé en ce qu'on détruit par hydrolyse au moyen de ficine, de papaïne ou d'une autre protéase ayant une spécificité égale ou analogue pour le substrat, la matière potentiellement antigénique dans la matrice intercellulaire obtenue, réticulée par des liaisons amide et ester.

7. Procédé suivant la revendication 6, caractérisé en ce qu'on exécute l'hydrolyse au moyen de ficine.

8. Procédé suivant la revendication 6 ou 7, caractérisé en ce qu'on traite en outre au moyen d'un dialdéhyde la matrice obtenue, réticulée par des liaisons amide et ester et exempte d'antigène.

9. Procédé suivant la revendication 6 ou 7, caractérisé en ce qu'on soumet la matrice obtenue, réticulée par des liaisons amide et ester et exempte d'antigène, en outre à un nouveau traitement de réticulation par formation de liaisons amide entre des radicaux amino libres et les radicaux carboxyle d'acides dicarboxyliques, d'acides tricarboxyliques ou d'acides polycarboxyliques de la série aliphatique, cycloaliphatique, aromatique ou hétérocyclique.